(19) Europäisches Patentamt / European Patent Office / Office européen des brevets

(11) **EP 2 111 844 A1**

(12) **EUROPEAN PATENT APPLICATION**

(43) Date of publication:
**28.10.2009 Bulletin 2009/44**

(51) Int Cl.:
*A61K 8/19* (2006.01)    *A61K 8/41* (2006.01)
*A61K 8/69* (2006.01)    *A61Q 11/00* (2006.01)
*A61K 8/365* (2006.01)

(21) Application number: **08155126.9**

(22) Date of filing: **24.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Gaba International AG**
**4142 Münchenstein (CH)**

(72) Inventors:
• **Moya Argilagos, Dally**
**8006, Zürich (CH)**
• **Matur, Turan**
**4103, Bottmingen (CH)**
• **Scheffel, Cornelia**
**4147, Aesch (CH)**

(74) Representative: **Bohest AG**
**Postfach 160**
**4003 Basel (CH)**

(54) **Oral care composition comprising dissolved tin and fluoride**

(57)    An oral care composition comprising a liquid phase containing dissolved tin, 200 to 2000 ppm fluoride ions, based on the oral composition, and 5 to 60 % by weight, based on the oral care composition, of a $C_{(3-5)}$ sugar alcohol; **characterised in that** the content of dissolved tin [Sn] in the liquid phase is at least 1000 ppm, based on the composition, and that the composition comprises an organic acid and ammonium cations of the formula (I):

$$R\text{-}NH^+R_a\text{-}[(CH_2)_u\text{-}NH^+R_b]_v\text{-}R_c \cdot \qquad (I)$$

wherein R is a saturated or unsaturated straight-chain hydrocarbon residue of 10 to 20 carbon atoms, v is an integer from 0 to 1, u is an integer from 2 to 3 and $R_a$, $R_b$ and $R_c$ are independently selected from hydrogen and $-CH_2CH_2OH$. The composition is preferably a mouthrinse. These compositions are tested in the treatment or prevention of erosive tooth demineralisation caused by food acids.

## Description

Field of the invention

The present invention relates to the field of oral care compositions, in particular mouthrinses, and their use in treating or preventing erosive tooth demineralization in acidic media, brought about by food acids or endogeneous acids such as gastric juice.

There are three major sources for acids, which can cause tooth demineralization. The first source are the acids generated by cariogenic oral bacteria from food debris. These acids are carboxylic acids derived from the carbohydrates of the food debris that are metabolized by the oral bacteria. Such acids are rather weak, but act for extended periods on the teeth. The second source are the exogeneous food acids that are present in the foodstuffs themselves, in particular in fruits, fruit juices or in artificial softdrinks, or in salad dressings. The third source are endogeneous acids, in particular hydrochloric acid-containing gastric juice, which may come into contact with the teeth upon vomiting, such as in bulimia patients, or in reflux disease patients. These latter two types of acids are rather strong but act only for short times on the teeth. Tooth demineralisation by caused by the latter two types of acids is termed "erosive tooth demineralisation" and is not related to cariogenic oral bacteria. Since acid-containing softdrinks have enjoyed a rising popularity among consumers in the past time the problem of erosive tooth demineralisation by food acids has become more acute, and a marked percentage of the overall population is nowadays afflicted by it. Similarly, a rising number of (mainly female) patients are subject to bulimia. Erosive tooth demineralisation is believed to be largely irreversible and is not noticed by the afflicted subject for quite a long time. The pathological condition is thus often only diagnosed at a late stage, when it is already beyond treatment or cure.

Prior art

It has been known for a long time that fluoride ion, such as in the form of sodium fluoride, stannous fluoride, sodium hexafluorophosphate or amine fluoride, is beneficial in preventing tooth demineralisation. These fluorides are customarily administered in the form of oral care products such as toothpastes, dental gels or mouthrinses. In view of the toxicity of fluoride at higher levels the total fluoride concentration in oral care products is kept below a typical level of 1500 ppm.

The applicant of the present application marketed at the time of filing of this application a mouthrinse (meridol®), containing the amine hydrofluoride OLAFLUR in an amount corresponding to 125 ppm fluoride and stannous fluoride in an amount also corresponding to 125 ppm fluoride.

It is known that a concentrated aqueous solution (10% by weight) of stannous fluoride, when it acts for prolonged time in vitro on dental enamel, produces an insoluble precipitate on the enamel found to be $Sn_3F_3PO_4$ (Archs. Oral Biol. 16, p.241ff, 1971).

In a Ph.D. thesis by Anne Schürmann "Effekte unter-schiedlich dosierter lokaler Fluoridapplikationen auf die erosive Demineralisation von humanem Dentin in situ" at the Justus-Liebig-Universität in Giessen, Germany (2004) three oral care products marketed by the applicant of the instant application were tested for their efficacy against erosive tooth demineralisation by citric acid, namely a) meridol® toothpaste, containing OLAFLUR in an amount corresponding to 350 ppm fluoride and stannous fluoride in an amount corresponding to 1050 ppm fluoride; b) the above mentioned meridol® mouthrinse, and c) elmex® gelee containing OLAFLUR in an amount corresponding to 2500 ppm fluoride and sodium fluoride in an amount corresponding to 10000 ppm fluoride, but no stannous salts. Tested were the toothpaste a), the double combination a)+b) and the triple combination a)+b)+c). It was observed that the efficacy against erosive tooth demineralisation increased from a) to double combination a)+b) to triple combination a)+b)+c), which was attributed to the increasing amounts of administered fluoride.

US 5,004,597 A discloses in its examples oral care compositions comprising more than 1000 ppm stannous ions, fluoride and about 10 % by weight of glycerol. The compositions of this publication were devoid of amine fluoride and were not intended for treating or preventing erosive tooth demineralisation.

None of the above mentioned printed publications examined the long-term storage behaviour of the disclosed solutions.

In EP 0 026 539 A it was observed that amine hydrofluorides, when formulated together with stannous fluoride in oral care compositions such as a mouthrinse, stabilizes stannous ions against oxidation and precipitation. Some of its examples were oral care formulations with more than 1000 ppm stannous ion. This publication did, however, not examine the effects of its compositions on erosive tooth demineralisation.

In J. Dent. Res. 50/3, p. 531ff (1971) the efficacy of 0.01 M stannous fluoride solutions (corresponding to about 1180 ppm stannous ion) against erosive enamel demineralisation caused by acetic acid / acetate buffer of pH 4.0 was tested, also after aging up to 21 days, and also in presence of the complexing agents glycerol or tartaric acid. It was found that after 21 days storage the stannous fluoride solution containing one of these two complexing agents was less active in preventing erosive enamel demineralisation than the solution containing stannous fluoride alone.

In a recent publication (Caries Res. 42, pp. 2-7, 2008) stannous chloride solution (815 ppm total tin content) and stannous fluoride solution (809 ppm total tin content) were tested in vitro for the treatment of erosive tooth demineralisation by citric acid. After citric acid erosion and subsequent stannous fluoride treatment the teeth samples seemed even more mineralised than before the erosion test (see its figure 1). The assay for erosion was, however, by X-ray measurements; the absorption reduction caused by tooth demineralisation was compensated partially, or even overcompensated, by the intense absorption of traces of stannous salts deposited onto teeth, thus causing an error in the apparent remineralisation efficacies. Furthermore, since this study was in vitro, it did not consider the influence of the salivary pellicle present in vivo on the teeth on the efficacy of the test solutions analysed.

The present application seeks to provide novel oral care compositions with improved efficacy in the treatment or prevention of erosive tooth demineralisation caused by food acids or endogeneous acids such as gastric juice and which is stable upon prolonged storage.

Summary of the invention

The object set is achieved by an oral care composition comprising a liquid phase containing dissolved tin, 200 to 2000 ppm fluoride ions, based on the oral composition, and 5 to 60 % by weight, based on the oral care composition, of a $C_{(3-5)}$ sugar alcohol; **characterised in that** the content of dissolved tin [Sn] in the liquid phase is at least 1000 ppm, based on the composition, and that the composition comprises an organic acid and ammonium cations of the formula (I):

$$R\text{-}NH^+R_a\text{-}[(CH_2)_u\text{-}NH^+R_b]_v\text{-}R_c \qquad (I)$$

wherein R is a saturated or unsaturated straight-chain hydrocarbon residue of 10 to 20 carbon atoms, v is an integer from 0 to 1, u is an integer from 2 to 3 and $R_a$, $R_b$ and $R_c$ are independently selected from hydrogen and $-CH_2CH_2OH$. Preferred embodiments of the oral care composition are as in the dependent claims.

Detailed description of the invention

The oral care compositions of the invention contain a liquid phase. By "liquid" is understood in the context of the present application that the phase designated as liquid should have a dynamic viscosity at room temperature of not more than 1000 mPa·s. The liquid phase is preferably at least partially aqueous. Accordingly, the liquid phase may preferably comprise about 30% to about 80% by weight, based on the liquid phase, of water. A possible co-solvent is ethanol, in amounts of typically 5% to 15 % by weight, based on the liquid phase.

The term "dissolved tin" is intended to encompass all ionic or non-ionic tin species in the formal oxidation states +II and/or +IV and being dissolved in the liquid phase. Examples of such dissolved tin species are hydrated stannous ions, stannous hydroxide, soluble ionic or nonionic complexes of stannous and/or stannic ions with the dissolved $C_{(3-5)}$ sugar alcohol and/or the anionic conjugate base of the dissolved organic acid as ligands, and ionic hydroxo complexes of stannous and/or stannic ions. Preferably 75 mol% or more of the content of dissolved tin [Sn] is tin in the formal oxidation state +II.

The term "$C_{(3-5)}$ sugar alcohol" is intended to encompass all polyhydric alcohols with a total carbon atom number n of 3 to 5 and a molecular formula of $C_nH_{(2n+2)}O_n$. Preferably these sugar alcohols are acyclic and unbranched. Examples of the $C_{(3-5)}$ sugar alcohol are glycerol, erythritol, threitol, arabitol, xylitol and ribitol. More preferred are acyclic unbranched $C_{(3-4)}$ sugar alcohols, such as glycerol, erythritol and threitol, and particularly preferred is glycerol. A more preferred range of content for the dissolved $C_{(3-5)}$ sugar alcohol is 25 to 45 % by weight, based on the oral care composition. The $C_{(3-5)}$ sugar alcohol is preferably dissolved in the liquid phase.

The organic acid is preferably a carboxylic acid. It is preferably dissolved in the liquid phase of the composition. The term "dissolved" implies here that the acid be dissolved either as the free acid or as a pharmaceutically acceptable salt of its anionic conjugate base (whichever may be the case) in the liquid phase of physiologically acceptable pH. Preferred subgroups of organic acids are edible di- or tricarboxylic acids with 4 to 6 carbon atoms including the carboxylate carbon atoms, such as succinic, tartaric, citric, malic, fumaric and adipic acids; or edible α-hydroxy $C_{2-6}$ carboxylic acids such as glycolic, lactic, citric, tartaric or gluconic acids. If the organic acid is dissolved in the form of a pharmaceutically acceptable salt then the counter cation may be a metal cation, such as from an alkaline metal (such as sodium or potassium), from an earth alkaline metal (such as magnesium or calcium), or from zinc. As an alternative the counter cation may be an ammonium cation of the above formula (I).

The content of the organic acid is preferably in the range of 0.01 to 10 % by weight, preferably 0.05 to 3 % by weight, based on the composition, whereby the upper limit may be given by the solubility of its conjugate base salt in the liquid phase at physiologically acceptable pH. The total content of organic acids may be determined by acidifying a known aliquot of the oral care composition to about pH 0, extracting the free organic acids with an organic solvent such as ether, and analysing the extract by calibrated GC using the silyl esters derivates of the acids.

Preferably the content of dissolved tin [Sn] in the liquid phase is in the range of 1000 ppm to 3000 ppm, more preferably in the range of 1300 ppm to 2500 ppm, even more preferably in the range of 1700 ppm to 2200 ppm, and most preferably in the range of 1900 to 2100 ppm, based on the composition. The total content of dissolved tin may be determined using X-ray fluorescence (see example 13). The content of dissolved tin in the formal oxidation state +II may be determined potentiometrically (see example 14). The dissolved tin may preferably be derived from a pharmaceutically acceptable stannic ion salt added to the oral care formulation. Examples are stannous chloride, stannous fluoride, stannous hydroxide, stannous sulfate, with stannous chloride being preferred.

The combination of dissolved tin species, of which some are rather acidic, and the pharmaceutically acceptable salt of the organic acid, which is rather basic, may yield in the lquid phase of the oral composition a pH range which is physiologically acceptable for an oral care composition, such as typically about 3.0 to about 7.0, preferably about 4.0 to about 5.0, more preferably about 4.3 to about 4.6. If necessary the pH of the oral care composition may be adjusted to the desired value by adding acid (such as hydrochloric acid) or base (such as sodium hydroxide).

The fluoride content of the oral care compositions is from 200 to 2000 ppm, based on the composition, preferably from 500 to 1000 ppm. Preferably the fluoride is dissolved in the liquid phase of the composition. The fluoride content of the oral care formulation may be determined potentiometrically using a fluoride-selective electrode (see example 15). The fluoride may be added to the oral care composition in the form of any fluoride ion source customarily employed in oral care compositions, e.g. as stannous fluoride and/or sodium fluoride and/or as above mentioned amine fluoride.

In one preferred embodiment of the oral care compositions of the invention the content of fluoride ions in ppm, based on the composition, $[F^-]$, is in the range of $0.60[Sn] \geq [F^-] \geq 0.40[Sn]$, wherein $[Sn]$ has the above meaning. In another preferred embodiment the content of fluoride ions in ppm, based on the composition, $[F^-]$, is in the range of $0.30[Sn] \geq [F^-] \geq 0.20[Sn]$, provided that the content of dissolved tin, $[Sn]$, as described above, is then in the range of 1900 to 2200 ppm.

The ammonium cations of formula (I) are derived from corresponding amines which contain one or two basic nitrogen atoms and which are converted to the ammonium cations of formula (I) by adding an amount of an acid which is acceptable in an oral care composition, such as hydrochloric, hydrofluoric, carbonic, citric, lactic or gluconic acids, preferably hydrochloric or hydrofluoric acids, and most preferably hydrofluoric acid, in which latter case the acid addition salts are known as "amine hydrofluorides" or "amine fluorides". The content of ammonium cations is preferably in the range of 150 ppm to 1000 ppm, based on the composition. Preferably the ammonium cations of formula (I) are dissolved in the liquid phase of the composition. The determination of the content of ammonium cations of formula (I) may be done over their corresponding free amine bases using calibrated reverse phase HPLTC (see examples 16 and 17).

In the ammonium cations of formula (I) the residue R can have even or odd-numbered chain length. Residues R having an even-numbered chain length are preferred with regard to physiological acceptability. The residues may be saturated or mono-, di- or polyunsaturated, preferably mono-unsaturated. Examples of saturated hydrocarbon residues having an even-numbered chain length are decyl, dodecyl (lauryl), tetradecyl (myristyl), hexadecyl (cetyl, palmityl), octadecyl (stearyl) and eicosanyl. Examples of unsaturated residues having an even-numbered chain length are 9-cis-octadecen-1-yl (oleyl), 9-trans-octadecen-1-yl (elaidyl), cis,cis-9,12-octadecadien-1-yl (linolyl), cis,cis,cis-9,12,15-octadecatrien-1-yl (lino-lenyl) or 9-cis-eicosaen-1-yl (gadolyl). Preferred are even-numbered $C_{16}$-$C_{20}$alkyl or even-numbered $C_{16}$-$C_{20}$alkenyl. More preferred are those where R is $C_{18}$alkyl or $C_{18}$alkenyl, and most preferred is 9-cis-octadecen-1-yl (oleyl).

The acid addition salts mentioned above containing the ammonium cations of formula (I) may be prepared in all instances by reacting the corresponding free amine base R-$NR_a$-$[(CH_2)_u$-$NR_b]_v$-$R_c$, wherein all symbols have the same meaning as in claim 1, with the appropriate acid in one equivalent, or slightly more than one equivalent (such as 1.05 equivalent) hydronium per basic nitrogen atom present in the free amine base. If the free amine base is a pure compound, the number of basic nitrogen atoms is clear from the structural formula. If the amine base is, however, a mixture of compounds, then the number of basic nitrogen atoms may be determined by titration of a sample of such a mixture with perchloric acid in glacial acetic acid using a glass electrode.

The preparation of the free amine bases themselves is briefly described in the following sections i) to iii).

i) In the case where v is 0 and $R_a$, $R_c$ are hydrogen the amine base is simply a fatty amine R-$NH_2$, wherein R has the meaning of claim 1.

ii) In the case where v is 0 and at least one of $R_a$ and $R_c$ is -$CH_2CH_2OH$ the amine may be obtained by hydroxyethylation of a fatty amine R-$NH_2$, with R as defined in claim 1, with one equivalent ethylene oxide, which gives the amines with $R_a$ as H and $R_c$ as -$CH_2CH_2OH$; or with two equivalents of ethylene oxide, which gives the amines with $R_a$, $R_c$ as -$CH_2CH_2OH$.

iii) In the case where v is 1 and u is 2 or 3 they may be prepared by acylation of ethylene diamine or propylene diamine, respectively, with an acyl chloride R-COCl, wherein R has the same meaning as in claim 1, which gives firstly amides with $R_a$, $R_b$ and $R_c$ as hydrogen. Reaction of the non-acylated nitrogen atom of these with one equivalent ethylene oxide gives the corresponding amides with $R_a$, $R_b$ as hydrogen and $R_c$ as -$CH_2CH_2OH$; or with two equivalents of ethylene oxide the corresponding amides with $R_a$ as hydrogen and $R_b$, $R_c$ as - $CH_2CH_2OH$. To furthermore introduce $R_a$ as -$CH_2CH_2OH$ any of these amides may be reacted at the amide nitrogen (the one having $R_a$ connected to it) with bromoethanol in the presence of a strong base such as t-BuOK, optionally with beforehand protection of the hydroxyl groups with dihydropyran. Any of the amides so obtained may then be reduced to the corresponding amine with lithium aluminium hydride, optionally with beforehand protection of any present hydroxyls with dihydropyran. If after the acylation step the said reaction sequence is inverted (i.e. first alkylation with 1-bromoethanol/t-BuOK, then reaction with one equivalent of ethylene oxide), and then the reduction with lithium aluminium hydride is performed, then the amine bases with $R_a$, $R_b$ as -$CH_2CH_2OH$ and $R_c$ as hydrogen are accessible.

As already stated the ammonium cations of formula (I) are most preferably added to the oral care composition as amine hydrofluorides. The amine hydrofluoride where R is 9-octadecen-1-yl (oleyl), v is 0 and $R_a$, $R_c$ are hydrogen is known under the international non-proprietary name of DECTAFLUR. The amine hydrofluorides where v is 0, $R_a$ and $R_c$ are -$CH_2CH_2OH$ and R is octadecyl or 9-octadecen-1-yl are known from the examples of WO 98/22427 A. The latter one of these two is known under the international non-proprietary name of XIDECAFLUR. The amine fluoride where v is 1, u is 3, and $R_a$, $R_b$ and $R_c$ are -$CH_2CH_2OH$ is known under the international non-proprietary name of OLAFLUR. OLAFLUR, DECTAFLUR and XIDECAFLUR are the particularly preferred amine hydrofluorides, and most preferred is OLAFLUR.

The oral care compositions may also comprise chloride ions, preferably as dissolved ions in the liquid phase. A preferred range of the chloride content [Cl⁻] in ppm, based on the composition, is in the range 0.65[Sn] ≥ [Cl] ≥ 0.55 [Sn], preferably in the range 0.62[Sn] ≥ [Cl] ≥ 0.56[Sn], and most preferably is about 0.60[Sn]. The chloride content may be determined by potentiometric titration (see example 18). The chloride may be added for example as sodium chloride, calcium chloride or stannous chloride, with the latter being preferred.

The oral compositions of the invention are preferably devoid of copper, meaning that they comprise preferably less than 0.05 % by weight, more preferably less than 0.001 % by weight, based on the composition, of copper.

It is understood that the oral care composition of the present invention is electroneutral, i.e. the sum of the negative charges brought about by all anions present is equal to the sum of all cations present.

The oral care composition of the invention may be any such formulation, e.g. be a toothpaste, dental gel, mouthrinse and so on.

The compositions of the invention, when they are mouthrinses, are clear solutions essentially, preferably completely free of suspended or sedimented solids or from turbidity.

The compositions of the invention, in particular in the form of mouthrinses, are efficacious in the treatment or prevention, particularly the prevention of erosive tooth demineralisation caused by food acids (i.e. acids originating from foods) or by endogeneous acids such as gastric juice. As "food acids" are considered in the context of the present application in particular phosphoric, acetic, propionic, benzoic, carbonic, citric, malic, oxalic, lactic, pyruvic, succinic, tartaric, tannic, caffeotannic, ascorbic, gluconic, glucuronic and glucaric acids, pectin, hydrated sulfur dioxide, and amino acids; and any salts thereof still containing at least one hydrogen atom which is dissociable to at least 50 mol% in aqueous solution at a pH typical for human saliva (i.e. a pH about 5.6 to about 8.4). Particularly are understood as food acids such acids with a first pKa value of 3.0 or less (such as phosphoric and citric acids, hydrated sulfur dioxide and aspartic acid), and/or which can act as chelating ligands for calcium ions (such as lactic, tartaric, citric, malic and amino acids) and/or which form low-soluble calcium salts (such as oxalic, carbonic and phosphoric acids). As "low soluble calcium salts" are understood in the present application calcium salts with a solubility of less than 0.1 g / 100 ml water of pH 5.7 at ambient temperature and pressure and at 35 Pa partial pressure of carbon dioxide.

Further optional components in all types of oral care composition of the invention may be for instance:

- Flavourings and cooling flavours, such as coumarin, vanillin, ethereal oils (such as peppermint oil, spearmint oil, aniseed oil, menthol, anethol or citrus oil) or other essences (such as apple, eucalyptus or spearmint essence). These flavourings may be present in 0% to 0.5%, preferably 0.03% to 0.3% by weight, based on the oral care composition.
- Sweeteners, in particular artificial sweeteners such as saccharin, acesulfam, neotam, cyclamate or sucralose; natural high-intensity sweeteners such as thaumatin, stevioside or glycyrrhizin; or sugar alcohols different from the $C_{(3-5)}$ sugar alcohol, such as sorbitol, xylitol, maltitol or mannitol. These may be present in amounts of 0% to 0.2%, preferably 0.005% to 0.1% by weight, based on the composition.
- Antibacterials and/or preservatives, such as chlorhexidine, triclosan, quaternary ammonium compounds (such as benzalkonium chloride) or parabens (such as methyl or propyl paraben). The amount of antimicrobial agent in the oral care composition is typically from 0 to about 0.5%, preferably 0.05 to 0.1% by weight, based on the oral care composition.

- Emulsifiers or solubilisers, mainly in connection with abovementioned flavourings and/or antibacterials, which often are of low solubility in aqueous media. Examples of such emulsifiers are neutral surfactants (such as polyoxyethylene hydrogenated castor oil or fatty acids of sugars), anionic surfactants (such as sodium lauryl sulfate), cationic surfactants (such as the ammonium cations of formula (I)) or zwitterionic surfactants. These surfactants or solubilisers may be present in amounts of typically 0% to 2%, preferably 0.2% to 1.5% by weight, based on the oral care composition.
- Thixotropic agents, such as soluble grades of hydroxypropylmethylcellulose, hydroxyethylcellulose or mucins, in an amount effective to impart the oral care composition a thixotropic behaviour.
- Stabilisers, such as polyvinylpyrrolidone.

Further optional components for oral care compositions of the invention that have a solid phase, such as in particular toothpastes or dental gels, are abrasives, such as inorganic abrasives (e.g. silica, aluminium oxide, calcium carbonate, calcium phosphate, calcium pyrophosphate or stannous pyrophosphate) or organic abrasives (such as polyethylene, polyvinyl chloride, polystyrene, polycarbonate, copolymers from (meth)acrylates and other olefinic monomers, polyamides, urea-formaldehyde resins, melamine-formaldehyde resins, phenol-formaldehyde resins, cured, pulverised epoxy resins or polyesters).

The oral care compositions of the invention may be used to treat or prevent erosive tooth demineralisation in a subject in need of such treatment or prevention. For this application it is preferred that the oral care composition be a mouthrinse. The mouthrinse is preferably provided to the subject in need in the form of a package containing both the mouthrinse and a leaflet onto which the instruction is printed to use the mouthrinse typically once a day, in an amount of typically 5 to 30 ml, preferably about 10 to 20 ml, depending on its content of the essential four ions, with the instructions to the subject to rinse the oral cavity for a certain period of time which is typically about 10 seconds to 1 minute, preferably about 30 seconds, again depending on the content of the mouthrinse, thus bringing the subject's teeth in contact with the mouthrinse. After the rinsing the mouthrinse may be discarded without swallowing, and preferably no rinsing of the oral cavity with water is performed afterwards. Such an administration regime is similar to the administration regime for mouthrinses of the prior art.

The oral care compositions of the instant invention are efficacious against erosive tooth demineralisation despite the fact that they preferably contain fluoride in an amount relative to the tin content which is neither as in stannous fluoride itself nor as in $Sn_3F_3PO_4$ (see the introduction), such as $0.60[Sn] \geq [F^-] \geq 0.40[Sn]$. The oral care compositions of the invention are stable and thus remain clear and precipitate-free for prolonged storage time. The oral care compositions of the present invention cause no coloration of the teeth, nor irritation of the gums, despite the fact that they contain appreciably higher amounts of tin than the known meridol® mouthrinse marketed by the applicant himself.

The invention will now be further explained by the following non-limiting examples.

Examples 1-12: Mouthrinse formulations

In the following examples "AmF" or "AmF 297" denotes the amine hydrofluoride OLAFLUR. The amounts of all ingredients listed in the table are in percentages by weight, based on the overall mouthrinse.

| Example No. | 1 | 2 | 3 | 4 | 5 | 6 |
|---|---|---|---|---|---|---|
| Formulation No. | 298/39 | 298/100 | 298/103 | 298/228 | 298/226 | 298/195 |
| Added actives | 750 ppm F⁻ ex AmF, 750 ppm F⁻ ex NaF, Sn 2800 ppm | 500 ppm F⁻ ex AmF, 500 ppm F⁻ ex NaF, Sn 2100 ppm | 250 ppm F⁻ ex AmF, 750 ppm F⁻ ex NaF, Sn 1400 ppm | 500 ppm F⁻ ex AmF, 500 ppm F⁻ ex NaF, Sn 2100 ppm | 250 ppm F⁻ ex AmF, 1000 ppm F⁻ ex NaF, Sn 2100 ppm | 150 ppm F⁻ ex AmF, 850 ppm F⁻ ex NaF, Sn 2100 ppm |
| AmF solution | 5.357 | 3.571 | 1.786 | 3.571 | 1.786 | 1.072 |
| SnCl₂ dihydrate | 0.534 | 0.408 | 0.272 | 0.407 | 0.407 | 0.407 |
| NaF | 0.166 | 0.1105 | 0.166 | 0.1106 | 0.2211 | 0.188 |
| Zinc lactate | | 0.75 | 0.75 | | 1 | 0.75 |
| HCl 20% | | | 0.066 | 0.44 | 0.06 | 0.07 |
| KOH 20% | 0.1 | | | | | |

(continued)

| Example No. | 1 | 2 | 3 | 4 | 5 | 6 |
|---|---|---|---|---|---|---|
| Formulation No. | 298/39 | 298/100 | 298/103 | 298/228 | 298/226 | 298/195 |
| Tego betain F50 | | 1 | 1 | 0.8 | 0.8 | 1.4 |
| Cremophor RH 410 | | | | 1 | 0.5 | 0.2 |
| Aroma | | | | 0.44 | 0.22 | 0.14 |
| Sodium saccharin | | | | 0.06 | | 0.05 |
| Acesulfam K | | | | | 0.1 | |
| Glycerol | 30 | 35 | 30 | 30.5 | 26 | 39 |
| Deionised water | 63.843 | 59.1605 | 65.96 | 61.6714 | 68.9059 | 55.967 |
| Sodium lactate | | | | | | 0.75 |
| Sodium citrate | | | | 1 | | |
| Sodium-D-gluconate | | | | | | |
| Exemple No. | 7 | 8 | 9 | 10 | 11 | 12 |
| Formulation No. | 298/306 | 298/307 | 298/215 | 298/292 | 298/380 | 298/381 |
| Added actives | 150 ppm F⁻ ex AmF, 850 ppm F⁻ ex NaF, Sn 2100 ppm | 500 ppm F⁻ ex AmF, 500 ppm F⁻ ex NaF, Sn 1900 ppm | 150 ppm F⁻ ex AmF, 350 ppm F⁻ ex NaF, Sn 2100 ppm | 125 ppm F⁻ ex AmF, 375 ppm F⁻ ex NaF, Sn 2100 ppm | 150 ppm F⁻ ex AmF, 350 ppm F⁻ ex NaF, Sn 2100 ppm | 125 ppm F⁻ ex AmF, 375 ppm F⁻ ex NaF, Sn 2100 ppm |
| AmF solution | 1.072 | 3.571 | 1.072 | 0.893 | 1.072 | 0.893 |
| $SnCl_2$ dihydrate | 0.403 | 0.364 | 0.407 | 0.403 | 0.405 | 0.405 |
| NaF | 0.188 | 0.1105 | 0.0774 | 0.083 | 0.0774 | 0.083 |
| Zinc lactate | 0.75 | 0.75 | | 0.5 | | 0.5 |
| HCl 20% | 0.07 | 0.05 | | 0.03 | | 0.03 |
| KOH 20% | | | 0.15 | | 0.21 | |
| Tego betain F50 | 1.4 | 1 | 1 | 0.4 | 0.4 | 0.5 |
| Cremophor RH 410 | 0.2 | 0.3 | 0.25 | 0.2 | 0.2 | 0.45 |
| Aroma | 0.13 | 0.27 | 0.14 | 0.12 | 0.2 | 0.18 |
| Sodium saccharin | 0.055 | 0.1 | 0.05 | 0.035 | 0.12 | 0.0175 |
| Acesulfam K | | | | | | |
| Glycerol | 39 | 26 | 41 | 32 | 28 | 28 |

(continued)

| Exemple No. | 7 | 8 | 9 | 10 | 11 | 12 |
|---|---|---|---|---|---|---|
| Formulation No. | 298/306 | 298/307 | 298/215 | 298/292 | 298/380 | 298/381 |
| Deionised water | 55.987 | 67.4005 | 54.8536 | 63.676 | 68.315 | 67.2915 |
| Sodium lactate | 0.75 | | | 1.5 | | 1.5 |
| Sodium citrate | | | | | | |
| Sodium-D-gluconate | | | 1 | 0.15 | 1 | 0.15 |

Example 13: Determination of the total content of dissolved tin [Sn] by X-ray fluorescence

As the x-ray fluorescence spectrometer a Thermo Noran QuanX is used. Two solutions are measured:

Solution 1: 5 g of the oral care formulation is directly filled into a XRF-cup. The XRF-cup is then closed with a polyethylene foil with the appropriate closing ring and is followingly inserted into the autosampler of the instrument Solution 2 is as solution 1, but with a known amount of furthermore added stannous salt [ΔSn] in the range of 80% to 120% of the expected ppm value of [Sn] of the sample solution.

Solutions 1 and 2 are each irradiated for 600 seconds with x-ray at 50 kV excitation, using a copper filter, $K_{\alpha}$-line at 25.193 keV. The integrated area under the fluorescence intensity peak of solution 1 is taken as $A_1$ and the integrated area under the fluorescence intensity peak of solution 2 is taken as $A_2$.

The dissolved tin content in ppm based on the composition, [Sn], is obtained as

$$[\text{Sn}] = [\Delta\text{Sn}]\frac{A_2}{A_2 - A_1}$$

Example 14: Measurement of dissolved tin at formal oxidation state +II

A combined platinum electrode type 6.1204.310 of Metrohm, Switzerland, and a potentiometer Titrando 809 of Metrohm, Switzerland, are used. The calibration of the electrode is done according to the manual.

10.0000 g of the oral care composition are exactly weighed ($\pm$ 0.1 mg) in a 100 ml container and 40 ml water, 5 ml 32wt% HCl and a known aliquot v (in ml) of standard 0.05 M $KI_3$ solution is added, such that iodine is added in excess of the tin in formal oxidation state +II contained in the sample (a typical value for v is 5 ml).

The electrode is immersed into the sample solution and the remaining iodine not already reduced to I$^-$ by the tin in formal oxidation state +II is titrated back with standard 0.1 M $Na_2S_2O_3$ solution to the endpoint of the titration. The used amount of $Na_2S_2O_3$ solution in ml is taken as $v_1$.

The tin in formal oxidation state +II contained in the sample in ppm based on the oral composition, [Sn$^{+II}$], is obtained as

$$[\text{Sn}^{+II}] = 593.45\,(v - v_1)$$

Example 15: Potentiometric fluoride determination in an oral care formulation of the invention

A fluoride-selective electrode type 6.0502.150 of Metrohm, Switzerland, a pH/Ion-meter 692, Metrohm, Switzerland and an Ag/AgCl reference electrode type 6.0750.100, Metrohm, Switzerland are used.

A total ionic strength adjusted buffer (TISAB) is required and made as follows: A solution of 160 mg NaOH in 2 litres water is prepared (solution 1); 25 g 1,2-diaminocyclohexane-N,N,N',N'-tetraacetic acid, 290 g NaCl chloride and 285 ml glacial acetic acid are dissolved in 2 litres water (solution 2); then solutions 1 and 2 are mixed and filled up to 5 litres with water.

The calibration of the fluoride-selective electrode is performed according to the manual of the pH/Ion-meter.

1.0000 g $\pm$ 0.1 mg of the oral care composition are exactly weighed in a 50 ml plastic container and filled up with water to a weight of 20.0000 g $\pm$ 0.1 mg and 20 ml of above mentioned TISAB buffer are added. The fluoride-selective electrode and the reference electrode are immersed into the sample and the potential is read off after 5 minutes, according to the manual of the pH/Ion-meter. The fluoride concentration in ppm is calculated by multiplying the measured response-value by 40, as total dilution volume, and dividing by the weight of the oral care composition sample in g.

Example 16: Determination of ammonium cations of formula (I) with $R_a$, $R_c$ = hydrogen and v = 0, or with $R_b$, $R_c$ = hydrogen and v = 1, in an oral care composition of the invention

The determination is done using densitometric quantification on reverse phase HPTLC plates after staining with ninhydrine.

Procedure:

Ninhydrine solution: Dissolve 2 g of ninhydrine purum in 1000 ml of ethanol p.a. The solution has to be stored in a glass bottle at 4 °C (maximal storage time: 1 month).

A reference solution of the ammonium cation to be determined is prepared by dissolving an exactly known amount of the corresponding pure amine hydrofluoride in methanol p.a., to make a solution containing an exactly known content of the amine fluoride in the range of about 3000 ppm, based on the solution. This reference solution is designated in the following as R.

Sample solution: Accurately weigh (to within 0.1 mg) an amount M of approximately 1 g of the oral care composition in a 25 ml measuring flask and make up to volume with methanol p.a. Expose to ultrasonic radiation for about 20 minutes. This solution is designated as S.

The HPTLC plate is Silicagel 60 without fluorescence indicator, 10 x 20 cm (Merck no. 5626).

The reference solution and the sample solution are applied onto the HPTLC plate using an applicator Linomat IV (Camag, Switzerland) according to the following track scheme:

| Track No. | Solution | Amount applied ($\mu$l) |
|---|---|---|
| 1 | R | 2 |
| 2 | S | 10 |
| 3 | R | 4 |
| 4 | S | 10 |
| 5 | R | 6 |
| 6 | S | 10 |
| 7 | R | 8 |
| 8 | S | 10 |
| 9 | R | 10 |
| 10 | S | 10 |
| 11 | R | 2 |
| 12 | S | 10 |
| 13 | R | 4 |
| 14 | S | 10 |
| 15 | R | 6 |
| 16 | S | 10 |
| 17 | R | 8 |
| 18 | S | 10 |
| 19 | R | 10 |
| 20 | S | 10 |

Each track has an initial width on the plate of 4 mm; the initial distance between two tracks is 5 mm and the initial distance from one outermost track to the adjacent edge of the plate is 11 mm.

The plate is developed with ethanol : 25% aqueous ammonia 9:1 (v/v) as the eluent to a migration distance of about 6cm (under these conditions e.g. the ammonium cation of formula (I) with $R_a$, $R_c$ = hydrogen and R = 9-octadecen-1-yl migrates to an $R_f$ value of about 0.6). The plate is then immersed in the ninhydrine solution for 10 min and dried for 10 min at 100°C.

Calculation:

The areas of all developed spots are evaluated densitometrically with light of wavelength 480 nm using a TLC scanner 3 (CAMAG, Switzerland).

The areas obtained from tracks 1, 3, 5, 7 and 9 are used to obtain a first parabolically approximated calibration curve of area vs. amount of amine fluoride in $\mu$g. A second such calibration curve is obtained from tracks 11, 13, 15, 17 and 19.

The average area from sample tracks 2, 6, 10, 14 and 18 is converted to an amount [am1] amine fluoride in $\mu$g using the first calibration curve. The average area from sample tracks 4, 8, 12, 16 and 20 is similarly converted to an amount [am2] amine fluoride in $\mu$g using the second calibration curve.

The content of ammmonium cations of formula (I) I ppm, based on the oral care composition, [AM], is then obtained as

$$[AM] = \frac{1250([am1]+[am2])}{M} \times \frac{(MW-19(v+1))}{MW}$$

wherein M, [am1] and [am2] are as defined above, MW is the molecular weight of the pure amine fluoride used to prepare solution R, and v is as defined for formula (I).

Example 17: Determination of ammonium cations of formula (I) in an oral care composition of the invention using densitometric quantification.

The procedure of this example is applicable to all other ammonium cations of formula (I) not falling under the definitions given in the heading of example 14. This determination is done on reverse phase HPTLC plates after staining with with Berlin Blue.

Berlin Blue solution: Dissolve 4 g of of potassium hexacyanoferrate(III) p.a. in 150 ml distilled water and add 350 ml of acetone p.a. Dissolve separately 7.5 g iron(III)chloride hexahydrate p.a. in 500 ml ethanol p.a. Mix immediately prior to use 40 ml of each of the two solutions and 80 ml of ethanol p.a.

A reference solution of the ammonium cation to be determined is prepared by dissolving an exactly known amount of the corresponding pure amine hydrofluoride in methanol p.a., to make a solution containing an exactly known content of the amine fluoride in the range of about 500 ppm, based on the solution. This reference solution is designated as R.

Sample solution: Accurately weigh (to within 0.1 mg) an amount M of approximately 1 g of the oral care composition in a 100 ml measuring flask and make up to volume with methanol p.a. Expose to ultrasonic radiation for about 15 minutes. This solution is designated as S.

The HPTLC plate is Silicagel 60 without fluorescence indicator, 10 x 20 cm (Merck no. 5626)

The reference solution and the sample solution are applied onto the HPTLC plate using an applicator Linomat IV (Camag, Switzerland) according to the following track scheme:

| Track No. | Solution | Amount applied ($\mu$l) |
|---|---|---|
| 1 | R | 1 |
| 2 | S | 3 |
| 3 | R | 2 |
| 4 | S | 3 |
| 5 | R | 3 |
| 6 | S | 3 |

(continued)

| Track No. | Solution | Amount applied (μl) |
|---|---|---|
| 7 | R | 4 |
| 8 | S | 3 |
| 9 | R | 5 |
| 10 | S | 3 |
| 11 | R | 1 |
| 12 | S | 3 |
| 13 | R | 2 |
| 14 | S | 3 |
| 15 | R | 3 |
| 16 | S | 3 |
| 17 | R | 4 |
| 18 | S | 3 |
| 19 | R | 5 |
| 20 | S | 3 |

Each track has an initial width on the plate of 4 mm; the initial distance between two tracks is 5 mm and the initial distance from one outermost track to the adjacent edge of the plate is 11 mm.

The plate is developed with n-pentanol : ethanol : diethyl ether : 25% aqueous ammonia 3:3:3:1 (v/v/v/v) as the eluent to a migration distance of about 6cm (under these conditions e.g. the ammonium cation of formula (I) with $R_a$, $R_b$, $R_c$ = 2-hydroxyethyl, R = 9-octadecen-1-yl, v = 1 and u = 3 migrates to an $R_f$ value of about 0.8). The plate is then immersed in the Berlin Blue solution for 10 min and dried for 10 min at 100°C.

Calculation:

The areas of all developed spots are evaluated densitometrically with light of wavelength 592 nm using a TLC scanner 3 (CAMAG, Switzerland).

The areas obtained from tracks 1, 3, 5, 7 and 9 are used to obtain a first parabolically approximated calibration curve of area vs. amount of amine fluoride in μg. A second such calibration curve is obtained from tracks 11, 13, 15, 17 and 19.

The average area from sample tracks 2, 6, 10, 14 and 18 is converted to an amount [am1] amine fluoride in μg using the first calibration curve. The average area from sample tracks 4, 8, 12, 16 and 20 is similarly converted to an amount [am2] amine fluoride in μg using the second calibration curve.

The content of ammmonium cations of formula (I) I ppm, based on the oral care composition, [AM], is then obtained as

$$[AM] = \frac{100000([am1]+[am2])}{6M} \times \frac{(MW-19(v+1))}{MW}$$

wherein M, [am1] and [am2] are as defined above, MW is the molecular weight of the pure amine fluoride used to prepare solution R, and v is as defined for formula (I).

Example 18: Potentiometric chloride determination in an oral care composition of the invention

A combined silver/silver chloride electrode type 6.0350.100 of Metrohm, Switzerland, and a potentiometer Titrando 809 of Metrohm, Switzerland, are used. The calibration of the electrode is done according to the manual.

1000 ± 0.1 mg of the oral care composition are exactly weighed in a 100 ml plastic container and 50 ml water and 2 ml 65 wt% nitric acid are added.

The electrode is immersed into the sample and the sample is titrated with standard 0.01 M silver nitrate solution to the endpoint of the titration. The used volume of silver nitrate solution in ml is taken as v.

The chloride contained in the sample in ppm based on the composition, [Cl⁻], is obtained as

$$[Cl^-] = 354.5 \text{ v}$$

Example 19: In situ tests with mouthrinses of the invention

The tests were carried out on enamel and dentin samples cut from extracted third molar teeth. The erosion tests were done ex situ, using a citric acid solution, and the treatment tests were done in situ, by letting probands carry the eroded samples in their mouth using a sample holder fixed to their jaw, and by using the mouthrinses of the invention. The test was carried out as a double-blind randomized test.

The enamel and dentin samples were prepared as follows: From the teeth were removed any remaining soft tissues and the roots. From the teeth surfaces on either the enamel or the dentin part were excised samples of about 1 mm thickness in the longitudinal direction of the tooth. The face of the samples representing the original, natural tooth surface was polished to yield a flat test surface of at least 3 x 3 mm using firstly grit paper of grain size 12 $\mu$m, then of 5 $\mu$m (the dentin or enamel, respectively, was removed to a maximum depth of about 250 $\mu$m). A total of 96 enamel and of 96 dentin samples was prepared in this way. The samples were stored until the tests in a refrigerator in a thymol solution, which was freshly prepared once a week.

The probands were eight persons having good oral conditions (no artificial dentures, no open carious lesions or obviously defect dental fixtures, no visible plaque). They had teeth salivary flow rates in the ranges of 0.25-0.35 ml/min (unstimulated) and 1.0-3.0 ml/min (stimulated); salivary buffering capacities in the ranges of 4.25-4.75 (unstimulated) and 5.75-6.5 (stimulated); and salivary pH values in the ranges of 6.5-6.9 (unstimulated) and 7.0-7.5 (stimulated).

The said sample holders for jaw insertion were individually modelled for each proband and had on each side three buccal supports for either two dentin samples and one enamel sample or for one dentin sample and two enamel samples. These sample holders were thus adapted to have three enamel and three dentine samples for each proband. The sample holders were disinfected prior to use by the proband by soaking for 30 minutes in 75 vol-% aqueous ethanol.

Each proband tested the mouthrinses of examples 7 and 8, the commercially available meridol® mouthrinse mentioned in the introduction and a placebo solution devoid of both stannous ions and of fluoride. He tested each of these in a 7-day test period, and tested them in a randomized, different order unknown to him. The procedure for each 7-day test period was as follows:

A) Before the test period:

1) A 5-day "wash-out" period was done in which the probands performed normal oral hygiene.

2) Half of the abovementioned polished test surface of the enamel or dentin samples was covered with a light-curable resin (Technovit 7230 VLC, Kulzer-Exact, Wehrheim, Germany) and the other half was carefully cleaned of any impurities. The covered part of the surface served as the reference surface for the profilometric determination of the demineralisation, whereas the uncovered part served as the demineralisation test area.

B) During the test period, for each day of the test period

1) At about 8:30 a.m. a first ex situ demineralisation treatment of the enamel or dentin samples inserted into the sample holders was done in at least 200 ml of 0.05 M citric acid solution for 5 minutes, then the sample holders were rinsed with running tap water for 1 min.

2) After that demineralisation an in situ oral treatment of 30 seconds with 10 ml of one of the mouthrinses according to the invention, with the sample holder mounted on the proband's jaw; the mouthrinse was then spitted out but no rinsing with water was done.

3) Five more ex situ demineralisation treatments at about 10:00 a.m., 11:30 a.m., 1:00 p.m., 2:30 p.m. and 4:00 p.m., under the same conditions as in the first demineralisation treatment, but without subsequent treatment with the mouthrinses of the invention.

The probands carried the sample holders on their jaws except during the meals or for personal oral hygiene; for these periods they stored the sample holders in a humid chamber. Before going to bed the probands cleaned the sample holders, but not the enamel or dentin samples, with a toothbrush without using a toothpaste, then immersed the sample holder for 5 minutes in a 0.1% by weight chlorhexidine gluconate solution.

C)After the test period
The enamel and dentin samples were taken out of the sample holders. The dentin samples were treated for 36 hours at 30°C with a solution of 15 units collagenase (*Clostridium histolyticum type VII*, Sigma Aldrich, St. Louis, USA) in 150 ml of a solution containing 0.4 $H_3PO_4$, 1.5 g KCl, 1 g $NaHCO_3$ and 0.2 g $CaCl_2$ per litre, in order to completely remove the dentine's organic matrix, which is prone to disturb the outcome of the subsequent profilometry. The samples were then numbered, glued onto object slides and stored in a humid chamber until the profilometric determination of their demineralisation.

The profilometric determination of the demineralisation extent is a measurement of height difference between reference part and test part of the sample surface (see point A) of the description of the 7-day test period above). The height profiles of the samples were measured with a Perthometer S8P (Perthen Mahr, Goettingen, Germany) with a mechanical probe (FRW-750, Perthen Mahr, Goettingen, Germany) for dentin samples and with an optical probe (Rodenstock, Munich, Germany) for enamel samples. The object slides with the samples glued onto them were fixed onto the xy-table of the profilometer with a mouldable fixing mass. For each of the samples three profilometries were run. The profilometries were evaluated using a special software (Perthometer Concept 4.0, Perthen Mahr, Goettingen, Germany). With this software two heights were determined by linear regression, one of them from the height profile found on the reference part of the sample and the other one from the height profile of the test area of the sample, whereby for both height profiles a border area up to a distance of 0.2 mm from the border line between reference and test areas was disregarded. The height difference between the centre points of the two linear regression lines in micrometers, averaged from the three runs for each sample, was considered as the extent of demineralisation of that sample. As the extent of dentin demineralisation of a proband was considered the average of the said height differences from the three dentin samples he was carrying during the test; as the extent of enamel demineralisation of a proband was considered the average of the said height differences from the three enamel samples he was carrying during the test.
The obtained data were checked for sufficient normal distribution (Kolmogorov-Smirnov test). The comparison of the results of all probands for each of the tested solution was done by simple variation analysis (ANOVA) with the posthoc test according to Tukey. The following results were obtained for the four solutions tested:

| mouthrinse | extent of demineralisation on enamel (micrometres) | extent of demineralisation on dentin (micrometres) |
|---|---|---|
| example 7 | 11.0 $\pm$ 5.7 | 26.4 $\pm$ 11.9 |
| example 8 | 9.7 $\pm$ 4.1 | 26.2 $\pm$ 6.7 |
| meridol® | 24.5 $\pm$ 14.4 | 32.8 $\pm$ 9.6 |
| Placebo | 54.8 $\pm$ 8.6 | 48.5 $\pm$ 13.0 |

These results show that the inventive mouthrinses have particularly on enamel an efficacy in preventing demineralisation which is about 2.5 times the efficacy of the commercial meridol® mouthrinse.
Furthermore, in none of the probands a dry mouth sensation occurred, nor any reddening of the gums or efflorescence of the proband's own teeth nor of the teeth samples were observed. In particular no significant tainting of neither the proband's own teeth nor of the samples was observed, despite the increased amounts of stannous ions as compared to the meridol® solution.
Example 20: In situ test with the mouthrinse of example 8
The mouthrinse of example 8 and a solution containing only NaF in an amount corresponding to 1000 ppm fluoride were tested and evaluated in a similar setup as described in example 17, but with 20 probands and using 180 enamel samples and 180 dentin samples. The following demineralisation results were obtained:

| | extent of demineralisation on enamel (micrometres) | extent of demineralisation on dentin (micrometres) |
|---|---|---|
| mouthrinse of example 8 | 9,2 $\pm$ 3,4 | 23,9 $\pm$ 6,4 |
| solution with 1000 ppm fluoride as | 24,2 $\pm$ 9,2 | 34,1 $\pm$ 9,3 |
| NaF | | |

It can be seen that the mouthrinse of example 8, containing a combination of stannous fluoride and amine fluoride amounting to a total of 1000 ppm fluoride, is much more effective particularly on enamel than the solution containing only NaF corresponding to 1000 ppm fluoride.

**Claims**

1. An oral care composition comprising a liquid phase containing dissolved tin, 200 to 2000 ppm fluoride ions, based on the oral composition, and 5 to 60 % by weight, based on the oral care composition, of a $C_{(3-5)}$ sugar alcohol; **characterised in that** the content of dissolved tin [Sn] in the liquid phase is at least 1000 ppm, based on the composition, and that the composition comprises an organic acid and ammonium cations of the formula (I):

$$R-NH^+R_a-[(CH_2)_u-NH^+R_b]_v-R_c \qquad \cdot (I)$$

   wherein R is a saturated or unsaturated straight-chain hydrocarbon residue of 10 to 20 carbon atoms, v is an integer from 0 to 1, u is an integer from 2 to 3 and $R_a$, $R_b$ and $R_c$ are independently selected from hydrogen and $-CH_2CH_2OH$.

2. The composition of claim 1, **characterised in that** the organic acid is a carboxylic acid.

3. The composition of claim 1 or 2, **characterised in that** the content of organic acid is 0.01 to 10 % by weight, based on the composition.

4. The composition of one of claims 1 to 3, **characterised in that** the total content of dissolved tin [Sn] in the liquid phase is in the range of 1000 ppm to 3000 ppm, based on the composition.

5. The composition of claim 4, **characterised in that** the total content of dissolved tin [Sn] in the liquid phase is in the range of 1300 ppm to 2500 ppm.

6. The composition of one of claims 1 to 5, **characterised in that** the content of ammonium cations of formula (I) is in the range of 1500 ppm to 10000 ppm, based on the composition.

7. The composition of one of claims 1 to 6, **characterised in that** in formula (I) R is $C_{16}$-$C_{20}$alkyl or $C_{16}$-$C_{20}$alkenyl.

8. The composition of claim 7, **characterised in that** R is $C_{18}$alkyl or $C_{18}$alkenyl.

9. The composition of one of claims 1 to 8, **characterised in that** in formula (I) either

   i) v is 0 and $R_a$, $R_c$ are hydrogen;
   ii) v is 0 and $R_a$, $R_c$ are $-CH_2CH_2OH$; or
   iii) v is 1, u is 3, and $R_a$, $R_b$, $R_c$ are $-CH_2CH_2OH$.

10. The composition of one of claims 1 to 9, **characterised in that** it consists of the liquid phase.

11. The composition of claim 10, **characterised in that** it is a mouthrinse.

12. The composition of one of claims 1 to 9, **characterised in that** it consists of the liquid phase and of 5 to 60 % by weight, based on the composition, of a solid phase being dispersed in the liquid phase.

13. The composition of claim 12, **characterised in that** it is a toothpaste or dental gel.

14. The composition of one of claims 1 to 13, for use in the treatment or prevention of erosive tooth demineralisation caused by food acids or endogeneous acids.

15. The composition of claim 14, wherein the use is for the prevention of erosive tooth demineralisation.

16. The composition of claim 14 or 15, wherein the food acids are acids with a first pKa value of 3.0 or less and/or are acids with chelating ability for calcium ions and/or which form low-soluble calcium salts, or the endogeneous acid is gastric juice.

**17.** A method of treating or preventing erosive tooth demineralisation caused by food acids or endogeneous acids in a subject in need of such treatment or prevention, wherein the subject's teeth are brought in contact with a composition according to one of claims 1 to 14 in an amount which is effective to treat or prevent such erosive tooth demineralisation.

**18.** The method of claim 17, which is for the prevention of erosive tooth demineralisation.

**19.** The method of claim 17, wherein the food acids are acids with a first pKa value of 3.0 or less and/or are acids with chelating ability for calcium ions and/or which form low-soluble calcium salts, or the endogeneous acid is gastric juice.

**20.** The method of claim 17, wherein the subject's teeth are brought in contact with the composition for a period of time in the range of 10 seconds to 1 minute.

**European Patent Office**

**EUROPEAN SEARCH REPORT**

Application Number

EP 08 15 5126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 5 395 241 A (KANDELMAN DANIEL [CA]) 7 March 1995 (1995-03-07) * claims 1,4,8 * | 1-20 | INV. A61K8/19 A61K8/41 A61K8/69 A61Q11/00 A61K8/365 |
| Y | US 2003/053963 A1 (PELLICANO JOSEPH J [US] ET AL) 20 March 2003 (2003-03-20) * paragraphs [0002], [0006]; claims 1,2,7,9,10,12 * | 1-20 | |
| Y | US 3 914 404 A (LANGER HORST G) 21 October 1975 (1975-10-21) * column 1, line 14 - column 2, line 18; claims; examples * | 1-20 | |
| Y | US 2007/025928 A1 (GLANDORF WILLIAM M [US] ET AL) 1 February 2007 (2007-02-01) * paragraph [0002]; claims 1,10; examples * | 1-20 | |
| Y | WO 94/27565 A (HAWE NEOS DENTAL [CH]; BAFFELLI GIANNI [CH]; VON WEISSENFLUH BEAT [CH]) 8 December 1994 (1994-12-08) * page 1, lines 6-14; claim 1 * | 1-20 | TECHNICAL FIELDS SEARCHED (IPC) A61K A61Q |
| Y,D | EP 0 026 539 A (GABA INTERNATIONAL AG [CH]) 8 April 1981 (1981-04-08) * page 11, lines 1-16; claims; examples * * page 16, line 1 - page 17, line 4 * | 1-20 | |
| Y,D | US 5 004 597 A (MAJETI SATYANARAYANA [US] ET AL) 2 April 1991 (1991-04-02) * column 2, lines 20-65; examples I-X,XIV,XV * | 1-20 | |

-/--

The present search report has been drawn up for all claims

| Place of search | Date of completion of the search | Examiner |
|---|---|---|
| Munich | 18 September 2008 | Pregetter, Magdalena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 08 15 5126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 98/22427 A (GABA INTERNATIONAL AG [CH]; HECKENDORN RENE [CH]) 28 May 1998 (1998-05-28) * page 1, line 4 - page 2, line 10; claims; examples 4,6,11,12 * * page 3, lines 6-9 * ----- | 1-20 | |
| Y | US 4 828 822 A (MUEHLEMANN HANS R [CH] ET AL) 9 May 1989 (1989-05-09) * claims; examples * ----- | 1-10 | |

TECHNICAL FIELDS
SEARCHED     (IPC)

The present search report has been drawn up for all claims

| Place of search | Date of completion of the search | Examiner |
|---|---|---|
| Munich | 18 September 2008 | Pregetter, Magdalena |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 08 15 5126

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2008

| Patent document cited in search report | | Publication date | Patent family member(s) | | Publication date |
|---|---|---|---|---|---|
| US 5395241 | A | 07-03-1995 | CA | 2070084 A1 | 22-08-1993 |
| | | | FR | 2687571 A1 | 27-08-1993 |
| US 2003053963 | A1 | 20-03-2003 | NONE | | |
| US 3914404 | A | 21-10-1975 | NONE | | |
| US 2007025928 | A1 | 01-02-2007 | NONE | | |
| WO 9427565 | A | 08-12-1994 | EP | 0675707 A1 | 11-10-1995 |
| EP 0026539 | A | 08-04-1981 | CA | 1141300 A1 | 15-02-1983 |
| | | | DE | 3069906 D1 | 14-02-1985 |
| | | | DK | 414580 A | 03-04-1981 |
| | | | FI | 803124 A | 03-04-1981 |
| | | | NO | 802906 A | 03-04-1981 |
| | | | US | 4828822 A | 09-05-1989 |
| US 5004597 | A | 02-04-1991 | NONE | | |
| WO 9822427 | A | 28-05-1998 | AT | 207873 T | 15-11-2001 |
| | | | AU | 4860597 A | 10-06-1998 |
| | | | BG | 103406 A | 31-03-2000 |
| | | | CA | 2272106 A1 | 28-05-1998 |
| | | | CZ | 9901659 A3 | 17-11-1999 |
| | | | DE | 59705220 D1 | 06-12-2001 |
| | | | DK | 944579 T3 | 07-01-2002 |
| | | | EE | 9900197 A | 15-12-1999 |
| | | | EP | 0944579 A1 | 29-09-1999 |
| | | | ES | 2165029 T3 | 01-03-2002 |
| | | | HR | 970622 A2 | 31-08-1998 |
| | | | HU | 0000377 A2 | 28-06-2000 |
| | | | IL | 129805 A | 23-05-2002 |
| | | | JP | 2000511930 T | 12-09-2000 |
| | | | KR | 20000053338 A | 25-08-2000 |
| | | | NO | 992308 A | 12-05-1999 |
| | | | PL | 333377 A1 | 06-12-1999 |
| | | | PT | 944579 T | 28-03-2002 |
| | | | SK | 63099 A3 | 08-11-1999 |
| | | | TR | 9901089 T2 | 21-09-1999 |
| | | | US | 2001006622 A1 | 05-07-2001 |
| | | | ZA | 9710381 A | 10-06-1998 |
| US 4828822 | A | 09-05-1989 | CA | 1141300 A1 | 15-02-1983 |
| | | | DE | 3069906 D1 | 14-02-1985 |
| | | | DK | 414580 A | 03-04-1981 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 08 15 5126

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2008

| Patent document cited in search report | Publication date | Patent family member(s) | | Publication date |
|---|---|---|---|---|
| US 4828822 A | | EP 0026539 A2 | | 08-04-1981 |
| | | FI 803124 A | | 03-04-1981 |
| | | NO 802906 A | | 03-04-1981 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5004597 A
- EP 0026539 A
- WO 9822427 A

**Non-patent literature cited in the description**

- *Archs. Oral Biol.,* 1971, vol. 16, 241ff
- **Anne Schürmann.** Effekte unter-schiedlich dosierter lokaler Fluoridapplikationen auf die erosive Demineralisation von humanem Dentin in situ. *Justus-Liebig-Universität in Giessen,* 2004
- *J. Dent. Res.,* 1971, vol. 50 (3), 531ff
- *Caries Res.,* 2008, vol. 42, 2-7